Europäisches Patentamt

European Patent Office

Office européen des brevets

(19)

(11) Numéro de publication : **0 626 400 A1**

# (12) DEMANDE DE BREVET EUROPEEN

(21) Numéro de dépôt : 94401099.0

(22) Date de dépôt : 18.05.94

(51) Int. Cl.⁵ : **C08G 18/65,** C08G 18/73, C08G 18/75, C08G 18/79, A61L 27/00

(30) Priorité : 18.05.93 FR 9306254

(43) Date de publication de la demande :
30.11.94 Bulletin 94/48

(84) Etats contractants désignés :
AT BE CH DE ES FR GB IT LI NL SE

(71) Demandeur : **Garcia, Alain**
**12 rue Jean Mermoz**
**F-33138 Lanton (FR)**

(72) Inventeur : **Garcia, Alain**
**12 rue Jean Mermoz**
**F-33138 Lanton (FR)**

(74) Mandataire : **Warcoin, Jacques**
**Cabinet Régimbeau,**
**26, avenue Kléber**
**F-75116 Paris (FR)**

(54) **Composition polymérisable à usage chirurgical ou dentaire, procédé de préparation d'une telle composition et implant dentaire.**

(57)    L'invention concerne une composition polymérisable conduisant à un polyuréthanne utilisé pour combler des cavités biologiques.

Le polyuréthanne est fabriqué par un procédé global, en une seule étape, à partir d'un oligomère diol saturé aliphatique ou cycloaliphatique de masse moléculaire comprise entre 500 et 2000, d'un triisocyanate organique aliphatique de masse moléculaire inférieure à 1000 et d'un polyol court saturé aliphatique ayant 2 à 10 atomes de carbone.

Le mélange polymérisable présente des caractéristiques de polymérisation permettant d'obtenir à 37°C, un degré d'avancement de la réaction comparable à celui obtenu à une température élevée.

La résistance mécanique et la biocompatibilité du polyuréthanne obtenu permettent son utilisation dans le domaine de la chirurgie orthopédique et dentaire pour des implantations à long terme.

EP 0 626 400 A1

Jouve, 18, rue Saint-Denis, 75001 PARIS

La présente invention est relative à une composition polymérisable à usage chirurgical ou dentaire.

La présente invention est également relative à un procédé de fabrication et de conservation d'un mélange polymérisable destiné à être injecté dans une enveloppe étanche introduite dans une cavité biologique. Ce mélange aboutit après la polymérisation, à la formation d'une masse compacte de polyuréthanne permettant la réalisation de structures de comblement de certaines cavités biologiques.

Des polymères peuvent être utilisés dans le domaine médical et chirurgical comme structures de comblement de certaines cavités biologiques. Ils ont le plus souvent un rôle de suppléance mécanique pour des structures osseuses ou articulaires. C'est le cas du comblement des cavités résultant de l'exérèse de tumeurs ou de formations pathologiques diverses au niveau du squelette. C'est le cas du comblement de la cavité résultant de la nucléotomie ou de toute autre technique ayant pour but la destruction du nucleus du disque intervertébral humain ou animal. Des polymères peuvent être utilisés pour combler des cavités pathologiques présentes sur ou dans la paroi des vaisseaux sanguins (anévrisme). Des polymères peuvent également servir de support intra-osseux pour des implants dentaires

Ces polymères doivent pouvoir être implantés à court terme ou à long terme (plus de 30 jours). Leur mise en oeuvre doit leur permettre de s'adapter à des cavités de formes complexes.

Le mélange polymérisable doit pouvoir être injecté au moyen d'une petite canule souple ou rigide reliée à une enveloppe étanche préalablement introduite dans la cavité grâce à un accès chirurgical ou percutané.

Le polymère obtenu doit pouvoir polymériser dans un temps compatible avec la durée de l'acte chirurgical. La polymérisation doit atteindre à 37°C, un stade d'avancement comparable à celui que l'on obtiendrait par chauffage à plus de 100°C. Il doit posséder des caractéristiques mécaniques adaptées aux contraintes physiologiques auxquelles la structure anatomique sera soumise. Les éléments constitutifs du mélange polymérisable comme le polyuréthanne obtenu doivent être facile à manipuler et ne pas présenter de propriétés susceptibles d'affecter nuisiblement la partie du corps avec laquelle ils sont en contact, en particulier l'os, les cartilages et les vaisseaux sanguins (biocompatibilité). Ils doivent être exempts de solvants ou autres produits nocifs. Il pourrait être intéressant d'introduire dans le mélange polymérisable des éléments chimiques radio-opaques, permettant de suivre par simple examen radiologique le devenir de la structure de comblement.

Il est connu d'utiliser des polymères pour combler des cavités biologiques.

Le brevet, U.S Patent, 3,030,951 décrit un procédé permettant de réparer les fractures par injection in-situ d'un mélange polymérisable à base de polyisocyanates aromatiques ou de toluène diisocyanate, de polyols, d'amines tertiaires et d'eau, qui aboutit à la formation d'une mousse rigide de polyuréthanne. Ce procédé est réalisé en deux temps, au contact direct avec le milieu biologique (sans l'introduction préalable d'une enveloppe). Cela est susceptible d'affecter nuisiblement la partie du corps avec laquelle il est en contact.

Le brevet français 83 10833, décrit un procédé de fabrication de structures à usage médical en polyuréthanne. Ce brevet concerne uniquement des polyuréthannes thermoplastiques fabriqués par un procédé global, destinés à être utilisés comme des structures orthopédiques externes.

Le brevet français 88 16184, que nous avons déposé précédemment, décrit une formulation de mélange polymérisable fabriqué par un procédé global. La formulation décrit un diisocyanate (Hylène W) qui présente des risques toxicologiques élevés et aboutit à la formation d'un polyuréthanne dont les propriétés mécaniques sont insuffisantes pour les applications énumérées ci-dessus.

On ne dispose pas à l'heure actuelle, à notre connaissance, d'un mélange polymérisable aboutissant à la formation d'un polyuréthanne présentant les caractéristiques de mise en oeuvre, de résistance mécanique et de biocompatibilité correspondant aux applications décrites ci-dessus. En particulier un polyuréthanne polymérisé dans un sac étanche, à la température de l'organisme, dont le degré d'avancement de la polymérisation soit sensiblement identique à celui obtenu à haute température, dont la résistance mécanique permet l'utilisation comme prothèse du nucleus du disque intervertébral et dont la biocompatibilité évite tout risque d'affecter nuisiblement la partie du corps avec laquelle il est en contact.

Il a maintenant été découvert grâce à une recherche méthodique, par le demandeur que de telles structures de comblement biologique peuvent être obtenues à partir de certains polyuréthannes possédant les propriétés souhaitées, énoncées précédemment. La présente invention a donc pour objet un procédé de fabrication de structures de comblement biologique caractérisées en ce qu'elles sont constituées essentiellement d'un polyuréthanne fabriqué par un procédé global, en une seule étape, à partir d'un oligomère diol saturé aliphatique ou cycloaliphatique, de masse moléculaire comprise entre 500 et 2000, d'un polyisocyanate organique, aliphatique ou cycloaliphatique et d'un polyol court saturé aliphatique ou cycloaliphatique ayant 2 à 10 atomes de carbone. De manière à établir un réseau moléculaire tridimensionnel capable de conférer au matériau les propriétés mécaniques recherchées dans les applications relevant de la chirurgie osseuse ou dentaire, on utilisera de préférence un triisocyanate avec un diol court. Le rapport entre le nombre de fonctions isocyanate et le nombre de fonctions alcool étant compris entre 0,9 et 1 et le nombre de moles dudit diol court et le nombre de moles dudit oligomère diol saturé étant compris entre 0,5 et 9.

2

L'invention est également relative à une composition polymérisable à usage chirurgical ou dentaire contenant, en mélange ou séparément, un oligomère diol saturé aliphatique ou cycloaliphatique de masse moléculaire comprise entre 500 et 2000, un polyol court saturé aliphatique ou cycloaliphatique, et un triisocyanate aliphatique ou cycloaliphatique de masse inférieure à 1000, le rapport entre le nombre de fonctions isocyanate et le nombre de fonctions alcool étant compris entre 0,9 et 1 et le nombre de moles dudit polyol court et le nombre de moles dudit oligomère diol saturé étant compris entre 0,5 et 9.

Selon une variante avantageuse, la composition peut être préparée à l'avance et être maintenue à une température inférieure à la température de polymérisation. Elle peut également contenir de façon indépendante les différents ingrédients.

Les oligomères diols saturés aliphatiques ou cycloaliphatiques de masse moléculaire comprise entre 500 et 2000 selon l'invention sont constitués par exemple de groupements polyesters (polyesterpolyol) ou polyether (polyetherpolyols).

Parmi les polyesterpolyols convenables on peut citer le "DESMOPHENE 1710" (marque déposée) fabriqué par "BAYER AG" (marque déposée) qui peut être utilisé dans les applications décrites ci-dessus et en particulier pour la suppléance mécanique des structures osseuses ou articulaires. Ce polyesterdiol linéaire a une masse moléculaire d'environ 1000 (poids-équivalent: 500).

Comme polyols courts saturés aliphatiques ou cycloaliphatiques ayant de 2 à 10 atomes de carbone utilisés dans nos préparations, on peut citer de façon non limitative l'éthylène glycol, le 1,3 propane diol, 1,4 butane diol, le diéthylène glycol, le 1,6 hexane diol, le 1,8 octane diol, le triméthyl 2,2,4-hexane diol, le 1,2 dicyclohexane diol, le cyclohexane diméthanol, le 1,3 cyclohexane diol, le 1,2,6 hexane triol.

Les triisocyanates organiques aliphatiques qui peuvent être utilisés sont des produits dérivés du diisocyanate 1-6 hexaméthylène comme le "DESMODUR N100" (marque déposée) ou le "DESMODUR N3300" (marque déposée), fabriqués par BAYER AG.(marque déposée) On retiendra les produits contenant la teneur la plus faible possible en diisocyanate 1-6 hexaméthylène.

Pour la structure de comblement destinée à polymérisée dans un sac étanche placé dans la cavité résultant de la nucléotomie selon la description faite plus haut, on retiendra un polyuréthanne fabriqué à partir de "DESMOPHENE 1710" (marque déposée) comme oligomère diol saturé, de "DESMODUR N3300" (marque déposée) comme triisocyanates organiques aliphatiques et de 1,4 butane diol comme diol court. L'ensemble formé par le polyuréthanne, le sac étanche et de son dispositif de fermeture constituant une prothèse de nucleus de disque intervertébral.

La masse de l'oligomère diol saturé ainsi que le rapport molaire entre diol court et l'oligomère diol saturé dépendent de l'application envisagée et en particulier de la résistance mécanique en compression et en fatigue. Le rapport entre le nombre de moles de diol court et le nombre de moles d'oligomère diol saturé peut varier entre 0,5 et 9 et de préférence entre 2 et 6 selon la masse de l'oligomère diol saturé et la résistance recherchée.

Dans le but d'obtenir un polyuréthanne où le nombre de fonctions isocyanate libres résiduelles est minimale, le rapport entre le nombre de fonctions isocyanate et le nombre de fonction alcool doit être légèrement inférieure à 1. Il est généralement compris entre 0,90 et 1.

Le procédé de fabrication des polyuréthannes utilisés dans la présente invention est un procédé classique, global, ne comprenant qu'une seule étape réactionnelle, c'est-à-dire que l'ensemble des réactifs (oligomères diol, triisocyanate, diol court) est mélangé en masse en une seule étape. Il s'agit d'un procédé suffisamment bien connu de l'homme de métier pour qu'il ne soit pas nécessaire d'en faire une description détaillée.

On mélange l'ensemble des réactifs (oligomères diol, triisocyanate, diol court), ils sont ensuite dégazés sous vide à 0,1 atmosphère pendant 10 à 20 minutes. La préparation est ensuite placée dans le dispositif d'injection puis conservé à une température inférieure à -10°C. Lors de son utilisation, le mélange polymérisable est porté à la température ambiante puis injecté dans le sac où la polymérisation va se réaliser à la température de l'organisme.

Pour la réalisation de l'invention, on utilisera de préférence le procédé global tel qu'il est défini précédemment.

Les structures de comblement des cavités biologiques sont réalisées en polyuréthanne présentant des ponts de réticulation, ce qui empêche les déformations avec l'élévation de la température, et leur procure une résistance mécanique suffisante au cours de leur utilisation. Pour la fixation d'implants dentaires dans le polymère le nombre de ponts de réticulation doit être maximum pour éviter l'arrachage de l'implant.

Les polyuréthannes selon l'invention ont subit avec succès les tests chimiques, mécaniques et de biocompatibilté en conformité avec leur utilisation dans les cavités organiques dans lesquelles ils sont destinés à être placés à long terme.

Selon une autre caractéristique, le mélange est conservé à une température inférieure à -10°C avant d'être injecté dans une enveloppe étanche dans laquelle se produit la réaction de polymérisation.

De telles structures de comblement des cavités biologiques peuvent être également réalisées avec les mêmes polyuréthannes que ceux décrits précédemment associés avec des charges, des pigments ou un support.

Le procédé de polymérisation ne nécessite aucun apport de chaleur extérieur autre que celui fournit par l'organisme vivant dans lequel le mélange polymérisable est placé.

La vitesse de la réaction entre l'isocyanate et les diols peut être augmentée ou diminuée de manière à adapter la durée totale de la polymérisation à celle de l'acte chirurgical. La vitesse peut-être modulée en augmentant ou en diminuant la concentration de certains catalyseurs bien connus de l'homme de métier.

L'invention a également pour objet un implant dentaire approprié à la mise en oeuvre de la composition polymérisable selon l'invention.

L'implant dentaire est caractérisé par une embase destinée à être fixée à l'entrée de la cavité osseuse, munie d'un conduit central, ladite embase étant associée à un sac étanche destiné à occuper la cavité osseuse et à contenir la composition polymérisable.

D'autres caractéristiques et avantages de cet implant dentaire ressortiront de la description qui suit. Cette description est purement illustrative et non limitative. Elle doit être lue en regard des dessins annexés sur lesquels :
- La figure 1 est une vue en coupe de l'implant dentaire fixé,
- La figure 2 est une vue en coupe de l'implant dentaire surmonté de la prothèse dentaire.

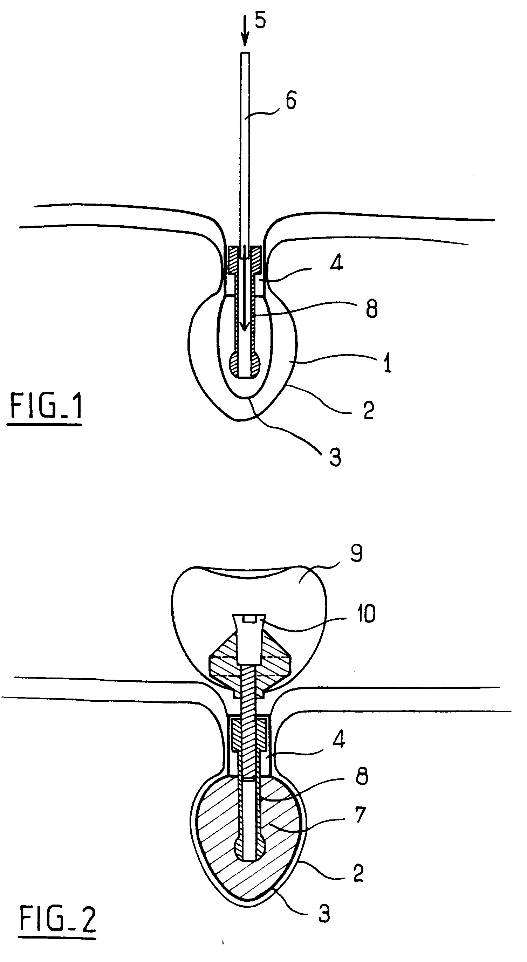

Selon les figures 1 et 2, le dispositif est constitué d'un implant en titane (4) muni d'une canule (8) permettant l'injection d'un mélange polymérisable (5) au moyen d'un tube externe (6) à l'intérieur d'un sac étanche (3) fixé sur l'implant (4). Le sac (3) est déployé dans la cavité (1) creusée dans l'os mandibulaire (2) sous l'effet de la pression d'injection du mélange polymérisable (5). Après la polymérisation du mélange (5), le polymère (7) obtenu stabilise l'implant (4) à l'intérieur de la cavité (1), s'oppose à son arrachage, et permet un amortissement des contraintes exercées sur l'implant (4). Dans un second temps, la prothèse dentaire (9) est fixée dans l'implant (4) grâce à la vis de fixation (10).

La présente invention est illustrée par l'exemple suivant, donné à titre non limitatif.

EXEMPLE : Réalisation d'une prothèse de nucléus de disque intervertébral

Les caractéristiques de fabrication sont rassemblées dans le tableau suivant :

| Matières premières | Caractéris-tiques des matières premières | Composition de la formulation | | Paramètres de formulation |
|---|---|---|---|---|
| | | molaire ramenée à 1 mole d'oligomère diol | pondérale en g/100 g | |
| Oligomère diol saturé "DESMOPHENE 1710" (marque déposée) | M = 1000 Poids-équivalent=500 OH-% = 3,4 | 1 Mole | 30,2 g | |
| Diol court: Butane diol 1-4 | M = 90 Poids-équivalent=45 eq OH/kg=22,2 | 4 Moles | 10,8 g | Nombre moles diol court= 4 nombre moles oligomère diol |
| Triisocyanate organique: "Desmodur N3300"(marque déposée) | M = 504 Poids-équivalent=195 NCO-% = 21,5 | 3,33 Moles | 58,9 g | nombre fonc-tions NCO= 1 nombre fonctions OH |
| Catalyseur : dibutyldilau-rate d'étain | - | - | - | $6.10^{-4}$ mmole.$g^{-1}$ du mélange |

tableau

La réaction est effectuée en masse dans un mélangeur approprié. La masse du mélange est de 10 g.

Mode opératoire.

1/ Préparation du mélange polymérisable.
- stérilisation des composants chimiques
- séchage sous vide du "DESMOPHENE 1710"(marque déposée) à 70°C durant 4 heures, puis introduction du butane diol 1-4.
- introduction du "DESMODUR 3300"(marque déposée) et du dibutyldilaurate d'étain puis agitation du mélange sous azote et dégazage sous vide pendant 15 minutes.
- introduction du mélange polymérisable dans le dispositif d'injection puis refroidissement à -20°C.
- conservation du mélange polymérisable à -20°C.

2/ Temps chirurgical.
- mise à la température ambiante du mélange polymérisable et de son contenant,
- nucléotomie du disque intervertébral pathologique,
- mise en place de l'enveloppe de polymérisation dans la cavité de nucléotomie,
- remplissage de l'enveloppe de polymérisation par injection du mélange polymérisable puis fermeture et retrait du dispositif de mise en place.
- polymérisation du mélange pendant 30 minutes à la température de l'organisme (37°C).

Caractéristiques du polyuréthanne obtenu.

- Propriétés optiques: Transparent et incolore.
- Propriétés mécaniques:
  Module d'Young: 22 Mpa.
  Coefficient de Poisson: $\nu = 0,49$
  Résistance en compression axiale: 45 Mpa.
  Résistance en cisaillement: supérieure à 2 Mpa.
- Propriétés en température.
  Température de transition vitreuse: TG = 1,1°C.
  Température de fusion = 170 °C.
- Propriétés biologiques: Biocompatibilité.

**Revendications**

1. Composition polymérisable à usage chirurgical ou dentaire contenant,en mélange ou séparément, un oligomère diol saturé aliphatique ou cycloaliphatique de masse moléculaire comprise entre 500 et 2000, un polyol court saturé aliphatique ou cycloaliphatique, et un triisocyanate aliphatique ou cycloaliphatique de masse inférieure à 1000, le rapport entre le nombre de fonctions isocyanate et le nombre de fonctions alcool étant compris entre 0,9 et 1 et le nombre de moles dudit polyol court et le nombre de moles dudit oligomère diol saturé étant compris entre 0,5 et 9.

2. Composition polymérisable à usage chirurgical ou dentaire selon la revendication 1, caractérisé en ce que l'oligomère diol saturé est le "DESMOPHENE 1710" (marque déposée).

3. Composition polymérisable à usage chirurgical ou dentaire selon la revendication 1, caractérisé en ce que l'oligomère diol saturé est du "DESMOPHENE 1710" (marque déposée), le triisocianate organique le "DESMODUR N 3300 ou N100" (marques déposées), le polyol court le 1,4 butane diol.

4. Composition polymérisable à usage chirurgical ou dentaire selon l'une quelconque des revendications 1 à 3, caractérisé en ce que le rapport entre le nombre de fonctions isocyanate et le nombre de fonctions alcool est compris entre 0,9 et 1 et le rapport entre le nombre de moles du diol court et le nombre de moles de l'oligomère diol saturé est compris entre 2 et 6.

5. Procédé de préparation d'un mélange polymérisable pour former des structures de comblement des cavités biologiques en polyuréthanne, fabriqué par un procédé global, en une seule étape, à partir d'un oligomère diol saturé aliphatique ou cycloaliphatique de masse moléculaire comprise entre 500 et 2000, d'un triisocyanate aliphatique ou cycloaliphatique de masse moléculaire inférieure à 1000 et un polyol court saturé aliphatique ou cycloaliphatique ayant 2 à 10 atomes de carbone, le rapport entre le nombre de fonctions isocyanate et le nombre de fonctions alcool étant compris entre 0,9 et 1 et le nombre de moles dudit polyol court et le nombre de mole dudit oligomère diol saturé étant compris entre 0,5 et 9.

6. Utilisation d'un mélange polymérisable selon l'une des revendications 1 à 4 pour la préparation d'une composition destinée au comblement des cavités biologiques, caractérisée en ce qu'il s'agit du comblement des cavités résultant de l'exérèse de tumeurs ou de formations pathologiques diverses au niveau du squelette.

7. Utilisation d'un mélange polymérisable selon l'une des revendications 1 à 4 pour la préparation d'une composition destinée au comblement des cavités biologiques, caractérisée en ce qu'il s'agit du comblement de la cavité résultant de la nucléotomie ou de toute autre technique ayant pour but la destruction du nucleus du disque intervertébral humain ou animal.

8. Utilisation d'un mélange polymérisable selon l'une des revendications 1 à 4 pour la préparation d'une composition destinée au comblement des cavités biologiques, caractérisée en ce qu'il s'agit du comblement de cavités pathologiques présentes sur ou dans la paroi des vaisseaux sanguins (anévrisme).

9. Utilisation d'un mélange polymérisable selon l'une des revendications 1 à 4 pour la préparation d'une composition destinée au comblement des cavités biologiques, caractérisée en ce que les structures de comblement obtenues sont destinées à servir de support pour des implants dentaires.

10. Implant dentaire selon l'une des revendications 1 à 4, caractérisé par une embase destinée à être fixée à l'entrée de la cavité osseuse, munie d'un conduit central, ladite embase étant associée à un sac étanche destiné à occuper la cavité osseuse et à contenir la composition polymérisable.

FIG.1

FIG_2

EP 0 626 400 A1

Office européen
des brevets

# RAPPORT DE RECHERCHE EUROPEENNE

Numero de la demande

EP 94 40 1099

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| X | PATENT ABSTRACTS OF JAPAN vol. 7, no. 277 (C-199) (1422) 9 Décembre 1983 & JP-A-58 157 814 (DAINIPPON INK) 20 Septembre 1983 * abrégé * | 1,5 | C08G18/65 C08G18/73 C08G18/75 C08G18/79 A61L27/00 |
| A | EP-A-0 383 596 (NIPPON POLYURETHANE INDUSTRY) * page 4, ligne 21 - ligne 27; revendication; exemple 1 * | 1,5 | |
| D,A | FR-A-2 639 823 (A. J. GARCIA) * en entier * | 1,3,4,10 | |
| A | WO-A-92 04390 (POLYMEDICA IND. INC.) * page 1, ligne 2 - page 3, ligne 3; revendications * | 1,4-10 | |

|  |  |  | DOMAINES TECHNIQUES RECHERCHES (Int.Cl.5) |
|---|---|---|---|
|  |  |  | A61L C08G |

Le présent rapport a été établi pour toutes les revendications

| Lieu de la recherche | Date d'achèvement de la recherche | Examinateur |
|---|---|---|
| BERLIN | 5 Septembre 1994 | Hoepfner, W |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)